(19) Europäisches Patentamt
European Patent Office
Office européen des brevets

(11) Veröffentlichungsnummer: **0 317 878 B1**

(12) # EUROPÄISCHE PATENTSCHRIFT

(45) Veröffentlichungstag der Patentschrift: **08.04.92**

(51) Int. Cl.$^5$: **A61K 37/02**, A61K 9/20, A61K 47/02, A61K 47/30

(21) Anmeldenummer: **88119014.4**

(22) Anmeldetag: **15.11.88**

(54) **Stabilisierte Arzneistoffe, Verfahren zu ihrer Herstellung sowie stabile Arzneizubereitungen.**

(30) Priorität: **24.11.87 DE 3739690**

(43) Veröffentlichungstag der Anmeldung:
**31.05.89 Patentblatt 89/22**

(45) Bekanntmachung des Hinweises auf die Patenterteilung:
**08.04.92 Patentblatt 92/15**

(84) Benannte Vertragsstaaten:
**AT BE CH DE ES FR GB GR IT LI LU NL SE**

(56) Entgegenhaltungen:
**EP-A- 0 050 191**
**EP-A- 0 158 927**
**EP-A- 0 280 999**
**US-A- 4 743 450**
**EP-A- 0 158 927**
**EP-A- 0 196 546**
**EP-A- 0 288 732**

**DICTIONNAIRE VIDAL, 1986, Seiten 1276-1277, O.V.P., Paris, FR**

**IDEM**

(73) Patentinhaber: **HOECHST AKTIENGESELLSCHAFT**
**Postfach 80 03 20**
**W-6230 Frankfurt am Main 80(DE)**

(72) Erfinder: **Fülberth, Werner, Dr.**
**Theodor-Storm-Strasse 11**
**W-6233 Kelkheim (Taunus)(DE)**
Erfinder: **Leeb, Richard, Dr.**
**Nonnbornstrasse 15**
**W-6233 Kelkheim (Taunus)(DE)**
Erfinder: **Radau, Manfred, Dr.**
**Altenhainer Strasse 61**
**W-6233 Kelkheim (Taunus)(DE)**
Erfinder: **Stammberger, Willi, Dr.**
**Sachsenring 15**
**W-6238 Hofheim am Taunus(DE)**

EP 0 317 878 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**CHEMICAL ABSTRACTS, Band 108, Nr. 8, 22. Februar 1988, Seite 402, Nr. 62328v, Columbus, Ohio, US; L. GU et al.: "Diketopiperazine formation, hydrolysis, and epimerization of the new dipeptide angiotensin-converting enzyme inhibitor RS-10085", & PHARM. RES. 1987, 4(5), 392-7**

**HAGERS HANDBUCH DER PHARMAZEUTISCHEN PRAXIS, Band 7A, Auflage 4, 1971, Seiten 499-502, Springer Verlag, Berlin, DE**

**INTERNATIONAL PHARMACEUTICAL ABSTRACTS, Band 3, Nr. 17, 15. September 1966, Spalte 1194, American Society of Hospital Pharmacists, Washington, DC, US; F. PRESCOTT: "PVP for tablet making", & DRUG COSMETIC IND. 97:497**

**Ritschel W.A.:DIE TABLETTE, 1966, Editio Cantor KG Aulendorf i.Würt, Seiten 46-47, 354-357, 366-369, 371-372, 211-212**

**Gu.L. et al. PHARMACEUTICAL RESEARCH, Band 4, Nr. 5, 1987, Seiten 392-397**

**Beschreibung**

Verbindungen der Formel I

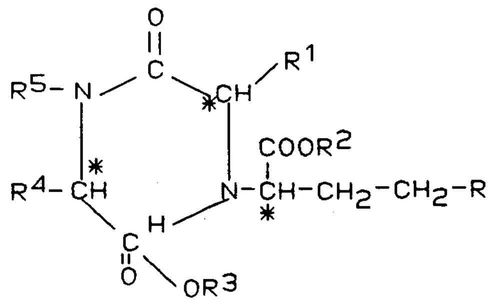

I

worin

R Wasserstoff, $C_1$-$C_4$-Alkyl oder Phenyl ist,

$R^1$ $C_1$-$C_4$-Alkyl oder

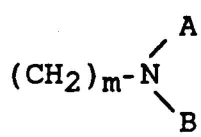

,

worin m 1, 2, 3 oder 4 ist und A und B gleich oder verschieden sind und Wasserstoff oder $C_1$-$C_4$-Alkyl bedeuten, darstellt,

$R^2$ Wasserstoff, $C_1$-$C_4$-Alkyl oder Benzyl ist

$R^3$ Wasserstoff oder $C_1$-$C_4$-Alkyl ist und

$R^4$ und $R^5$ zusammen mit den sie tragenden Atomen ein heterocyclisches, mono-, bi- oder tricyclisches hydriertes oder teilhydriertes Ringsystem mit 1 Stickstoffatom und 4 bis 15 Ringkohlenstoffatomen, das gegebenenfalls mono- oder disubstituiert ist mit $C_1$-$C_4$-Alkoxy, bedeuten,

stellen wertvolle Arzneimittel dar. Sie sind z.B. Hemmstoffe des Angiotensin-Converting-Enzyms (ACE) und können zur Bekämpfung des Bluthochdrucks verschiedener Genese eingesetzt werden. Auch eine nootrope Wirkung dieser Verbindungen wurde beschrieben (vgl. Deutsche Offenlegungsschrift 36 10 391 entspr. EP-A-0243645 bzw. US-Patentanmeldung Serial-Number 29905). Die Verbindungen der Formel I sind z.B. aus EP-A-79022 und EP-A-50800 bekannt; ferner sei auch auf die in der Deutschen Offenlegungsschrift 36 10 391 angegebenen Literaturstellen verwiesen.

Die Wirkstoffe der Formel I werden vorzugsweise oral verabreicht, fest Applikationsformen, wie z.B. Tabletten, Dragees oder Kapseln sind besonders geeignet.

Es wurde festgestellt, daß Wirkstoffe der Formel I, wie z.B. 2-[N-[(S)-1-Ethoxycarbonyl-3-phenylpropyl]-L-alanyl](1S,3S,5S)-2-azabicyclo[3.3.0]octan-3-carbonsäure (Ramipril) in pharmazeutischen Zubereitungen in Abhängigkeit von den engesetzten Hilfsstoffen, vom Herstellungsverfahren und von der Lagerung Tendenzen zur Instabilität zeigen.

Als Hauptzersetzungsprodukt läßt sich in pharmazeutischen Zubereitungen die durch Kondensation entstandene Diketopiperazin-Verbindung mit folgender Struktur II

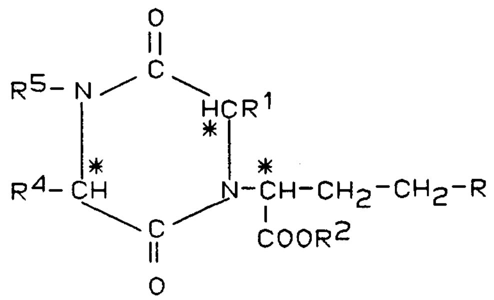

II

nachweisen. Das Hauptzersetzungsprodukt von Ramipril ist demnach des Diketopiperazin-Derivat der Formel IIa

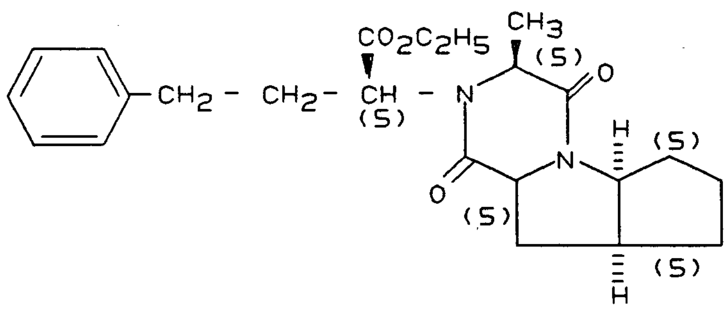

**IIa**

Es wurde gefunden, daß durch die Auswahl geeigneter Hilfsstoffe die Stabilität beeinflußt werden kann und daß eine wesentliche Zersetzungsursache der mit dem Herstellungsverfahren verbundene mechanische Streß ist, vor allem dann, wenn der Wirkstoff, z.B. Ramipril, im Gemisch mit Hilfsstoffen vorliegt.

In der folgenden tabellarisch zusammengefaßten Untersuchung wird der zersetzungsinduzierende Einfluß der mechanischen Belastung am Beispiel von Ramipril verdeutlicht.

Ramipril-Tabletten zu 2,5 mg/Einfluß des mechanischen Stresses auf die Stabilität.

| Diketopiperazin-Derivat vom Ramipril (%) | | | |
|---|---|---|---|
| Zeit und Art der Belastung | Tabletten 2,5 mg | Tabletten 2,5 mg | Kapseln 2,5 mg |
| 3 Mon. + 40°C | 13,6 | 7,6 | 4,0 |
| 6 Mon. + 40°C | 22,8 | 12,0 | 6,4 |
| Hersetllungsverfahren | Trockengranulation | Direktverpressung | Abfüllung des Pulvergemischs |
| Mechanischer Streß | hoch | mittel | niedrig |

Die Zusammensetzung der drei verglichenen Zubereitungen ist identisch und enthält folgende Hilfsstoffe: Mannit, mikrokristalline Zellulose, Na-stearylfumarat. Der Unterschied besteht allein in dem verarbeitungsbedingten Preßdruck (mechan. Streß).

Die Ergebnisse zeigen deutlich, daß der mechanische Streß ein wesentlicher, zersetzungsinduzierender Faktor ist.

Es wurde ferner gefunden, daß die Lagerungsbedingungen die Stabilität der Wirkstoffe der Formel I beeinflussen. Die Zersetzung wird bei ansteigender Temperatur und Feuchte und durch das Zusammenwirken beider Lagerungseinflüsse begünstigt.

Die Zersetzungsneigung z.B. von Ramipril in Zubereitungen, in denen alle genannten Einflußfaktoren zusammenwirken, ist im folgenden Vergleichstest zu erkennen:

Es wurde der Wirkstoffgehalt nach Belastung bestimmt von

a) der Wirksubstanz an sich; unverpreßt

b) von Ramipril-Tabletten, die mehrere Hilfsstoffe enthielten und einer mechanischen Belastung (Verpressen) ausgesetzt waren:

| Zeit und Art der Belastung | Gehalt bezogen auf den Ausgangswert | |
|---|---|---|
| | Ramipril-Wirksubstanz | Ramipril-Tabletten |
| 6 Monate + 40°C | 99 % | 56 % |
| 6 Monate + 40°C<br>80 % rel. F. | 96 % | < 20 % |

**Eingesetzte Tablettierhilfsstoffe**

Lactose-Monohydrat, Maisstärke, mikrokristalline Zellulose, Natriumstärkeglykolat, hochdisperses Siliciumdioxid, Talkum, Magnesiumstearat.

Die Ergebnisse zeigen deutlich, daß die unverpreßte Wirksubstanz unter den gewählten Prüfbedingungen eine gute Stabilität aufweist. Erste die Verpressung (mechan. Streß) mit allgemein üblichen Tablettierhilfsstoffen führt nach Temperatur- und vor allem Feuchtebelastung zu einem starken Abfall des Wirkstoffgehalts.

Die bevorzugte Zubereitungsform für die Wirkstoffe der Formel I ist wegen der individuellen Möglichkeit der Dosiseinstellung und besseren Patienten Compliance die Tablette. Die Zubereitungsformen sind wie die obigen Ergebnisse zeigen, extrem instabil, insbesondere durch das Zusammenwirken von

1. mechanischem Streß (Preßdruck)
2. Tablettierhilfsstoffen
3. Temperatur
4. Feuchtigkeit.

Während bei der Herstellung von Zubereitungen in gepreßter Form auf mechanischen Streß nicht verzichtet werden kann, wurde versucht, durch Änderungen der Hilfsstoffe zu stabilen Zubereitungen zu gelangen. Am Beispiel von Ramipril konnte durch gezielte Auswahl von Hilfsstoffen hinsichtlich ihrer Kompatibilität mit Ramipril die Rezeptur optimiert werden. Dies verdeutlicht der nachstehende Vergleich nach Streßbelastung.

| Zeit und Art der Belastung | Ramipril-Tabletten zu 1 mg Gehalt bezogen auf den Ausgangswert | |
|---|---|---|
| | Rezeptur I | Rezeptur II (optimierte Rezeptur) |
| 6 Monate 40 ° C | 56 % | 88,5 % |
| Hilfsstoffe | Lactose-Monohydrat, Maisstärke, mikrokristalline Zellulose, Na-Stärkeglykolat, hochdisperses Siliciumdioxid, Talkum, Magnesiumstearat | Mannit, mikrokristalline Zellulose, Na-Stearylfumarat |

Jedoch ist diese Maßnahme allein nicht ausreichend, die Tablettenzubereitung zu stabilisieren. Überraschenderweise wurde nun gefunden, daß eine Schutzumhüllung der zersetzungsanfälligen Ramipril-Substanz mit polymeren Filmbildnern der mechanischen Desaktivierung entgegenwirkt. Überraschend waren diese Befunde deshalb, weil schon geringe Umhüllungsmengen ausreichten, den Wirkstoff gegenüber mechanischer Belastung abzuschirmen.

Es ist bereits beschrieben worden, daß Arzneiwirkstoffe durch Mikroverkapselung mit Polymeren gegen Sauerstoff, Feuchtigkeit oder andere chemische Agenzien geschützt werden können (Hagers Handbuch der pharmazeutischen Praxis, Band 7A, Springer Verlag, Berlin, 499-502, 1971). Ebenso ist beschrieben worden, daß Tabletten z.B. zum Schutz gegen äußere Einflüsse wie Feuchtigkeit mit Polymerfilmen umhüllt werden (W.A. Ritschel, Die Tablette, 1966, Editio Cantor KG/Aulendorf i. Württ., Seiten 46-47, 211-212, 354-357, 366-369, 371-372). Entsprechend den Angaben in EP-A-0288732 kann die Freigabe eines ACE-Inhibitors aus Beadlets durch Umhüllung derselben mit einem Polymerfilm modifiziert werden.

Es wurde außerdem gefunden, daß man stabile, für die perorale Anwendung geeignete Tabletten erhält, wenn man der Wirksubstanz der Formel I einen Puffer zusetzt, der sicherstellt, daß der unter der Einwirkung von Luftfeuchtigkeit sich in der Zubereitung einstellende pH-Wert im schwach sauren bis schwach alkalischen Bereich (5,5 bis 8,0) liegt.

Entsprechend EP-A-0280999 wird die Instabilität von ACE-Inhibitoren in pharmazeutischen Präparaten durch Zusatz von Alkali- oder Erdalkalicarbonat und Zucker herabgesetzt. Die in Dictionnaire Vidal, 1986, O.V.P. Paris, 1276-1277 aufgeführte Enalapril-Zusammensetzung enthält u.a. Natriumhydrogencarbonat und Lactose.

Die Erfindung betrifft daher stabile, gepreßte pharmazeutische Zubereitung enthaltend eine Verbindung der Formel I oder deren physiologisch verträgliche Salze, dadurch gekennzeichnet, daß eine Verbindung der Formel I oder ein physiologisch verträgliches Salz einer Verbindung der Formel I zur Stabilisierung vor dem Verpressen

a) mit einer polymeren, physiologisch verträglichen Schutzhülle umhüllt wird oder

b) mit einem physiologisch verträglichen Puffer, der sicherstellt, daß sich in einer pharmazeutischen Zubereitung in Gegenwart von Feuchtigkeit ein pH-Wert im schwach sauren bis schwach alkalischen Bereich einstellt, vermischt wird, wobei Natriumhydrogencarbonat als Puffer ausgeschlossen ist, oder

c) mit einem physiologisch verträglichen Puffer, der sicherstellt, daß sich in einer pharmazeutischen Zubereitung in Gegenwart von Feuchtigkeit ein pH-Wert im schwach sauren bis schwach alkalischen Bereich einstellt, vermischt und mit einer polymeren, physiologisch verträglichen Schutzhülle umhüllt wird,

wobei im Falle der Stabilisierung gemäß b) mit Alkali- oder Erdalkalicarbonat die Zubereitung frei von Zucker ist, sowie ein Verfahren zu deren Herstellung.

Weitere Einzelheiten der erfindungsgemäßen Zubereitung und des Verfahrens zu deren Herstellung sind in den Patentansprüchen definiert.

Die Chiralitätszentren an den in Formel I mit einem Stern (*) markierten Kohlenstoffatomen haben vorzugsweise die S-Konfiguration.

Wirkstoffe der Formel I, worin R, $R^1$, $R^2$ und $R^3$ die folgenden Bedeutungen haben:

R : Methyl oder Phenyl,
$R^1$: Methyl oder $(CH_2)_4$-$NH_2$,
$R^2$: Wasserstoff oder Ethyl,
$R^3$: Wasserstoff und worin
$R^4$ und $R^5$ mit den sie tragenden Atomen vorzugsweise folgende Ringsysteme bilden

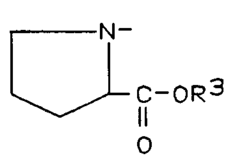
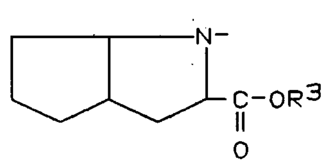
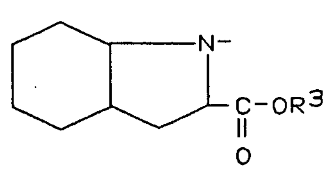
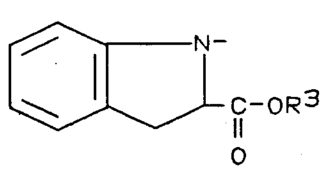
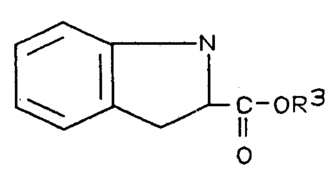

wobei $R^3$ vorzugsweise Wasserstoff ist, sind bevorzugt.

Als Wirkstoffe kommen insbesondere in Betracht:

Ramipril der Formel Ia

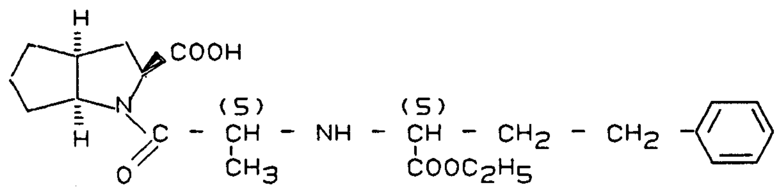

Ia

Enalapril der Formel Ib

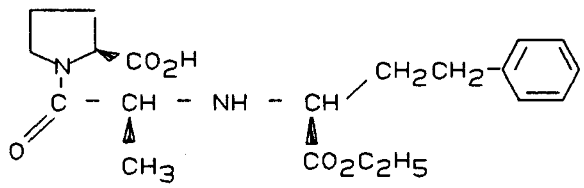

Ib

Perindopril der Formel Ic

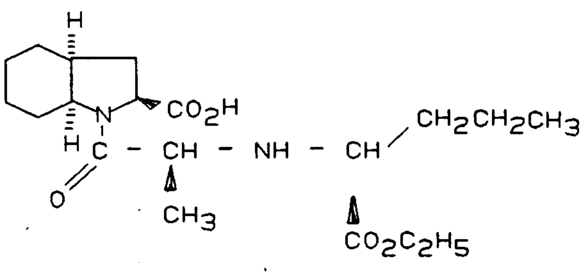

Ic

Indolapril der Formel Id

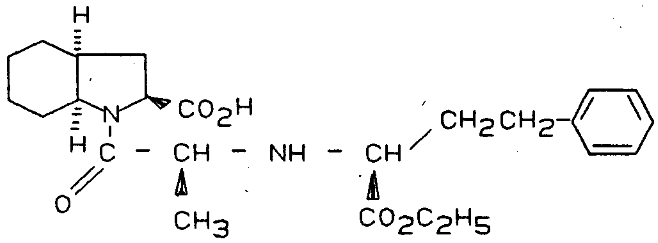

Id

Lisinopril der Formel Ie

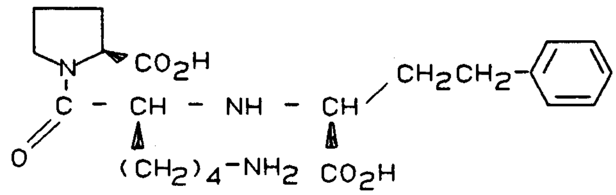

Ie

Quinapril der Formel If (X= H)
Alacepril der Formel If (X= 3,4-$OCH_3$)

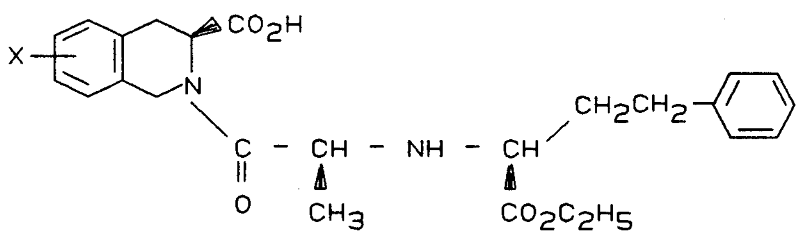

If

Trandolapril der Formel Ig

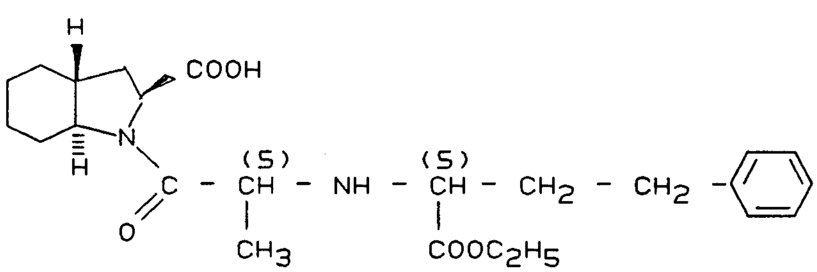

Ig

sowie

CGS 13928 C der Formel Ih

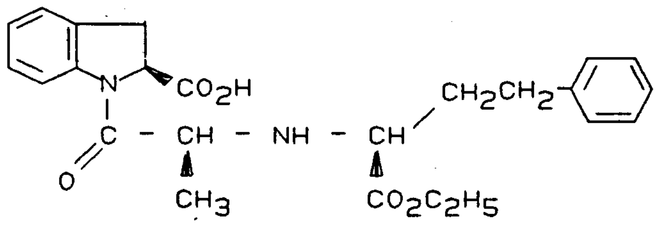

Ig

Wirksam sind bereits Schutzumhüllungen in Konzentrationen von 3 bis 25%, vorzugsweise 5 bis 15 % (Gewichtsprozent, bezogen auf den zu umhüllenden Wirkstoff). Es war nicht zu erwarten, daß selbst dünne Filmhüllen den Inhalt vor hohen mechanischen Belastungen, wie sie beim Tablettierprozess üblich sind (5 KN bis 30 KN), abschirmen können.

Weiterhin war überraschend, daß die zur Schutzumhüllung vorgesehenen Polymere als wäßrige Lösungen eingesetzt werden können, ohne daß die Stabilität negativ beeinflußt wird.

**Beispiele für die zur Schutzumhüllung in Frage kommenden Polymere**

Cellulosederivate, wie z.B. Hydroxypropylcellulose, Hydroxypropylmethylcellulose, Hydroxypropylmethylcellulosephthalat, Hydroxyethylcellulose, Ethylcellulose, Celluloseacetatphthalat, Celluloseacetat, Polyvinylacetatphthalat, Polyvinylpyrrolidon, kationische und anionische Polymerisate, Copolymerisat mit neutralem Charakter auf Basis von Poly(meth)acrylsäureestern (Eudragit® E, Eudragit® E 30 D), anionisches Polymerisat aus Methacrylsäure und Methacrylsäuremethylester (Eudragit® L oder S, Eudragit® L 30 D), Gelatine. Grundsätzlich kommen alle physiologisch verträglichen Polymere in Frage.

Die Schutzumhüllung kann durch Dispergieren von Wirksubstanz mit der Lösung oder Dispersion des Filmbildners in einem geeigneten Kneter, Mischer oder Mischer-Granulator vorgenommen werden. Die gleichmäßig benetzte Masse wird anschließend durch ein Sieb getrieben und getrocknet. Das getrocknete Granulat wird nochmals durch ein Sieb passiert und dann zur Herstellung von Kapseln oder Tabletten eingesetzt.

Eine besonders gleichmäßig Umhüllung gelingt in der Wirbelschicht. Die Wirkstoffpartikel werden im Luftstrom mit einer Lösung oder Dispersion des Polymeren besprüht und getrocknet.

Das umhüllte Wirkstoffgranulat kann nach dem Trocknungsprozeß direkt zur Abfüllung in Kapseln oder zur Herstellung von Tabletten verwendet werden.

Man kann aber auch beide Prozesse miteinander kombinieren, indem man zunächst im Kneter, Mischer oder Mischer-Granulator die Wirksubstanz mit der Polymerlösung oder -dispersion benetzt und durch anschließendes Granulieren zu gleichmäßigen Agglomeraten aufarbeiten, die dann in der Wirbelschicht abschließend mit der Polymerlösung oder -dispersion umhüllt werden.

Die nach dem erfindungsgemäßen Verfahren durch einen Schutzfilm stabilisierten Wirkstoffe können zu Kapseln oder gepreßten Darreichungsformen verarbeitet werden. Solche Präparate sind im Verlgeich zu Präparaten, die mit unbehandeltem Wirkstoff hergestellt werden, stabil. Dies läßt sich am besten am Beispiel von Tabletten erkennen, bei denen sich der mechanische Streß bei der Herstellung nach anschließender Temperaturbelastung stabilitätsmindernd bemerkbar macht.

Ein Stabilitätsvergleich mit einer Standardrezeptur ohne Schutzumhüllung ist der nachfolgenden Tabelle zu entnehmen.

Tabelle 1

| Ramipril-Tabletten zu 2,5 mg<br>Stabilitätsvergleich/stabilisierender Einfluß einer Schutzumhüllung<br>Belastungsart: 6 Monate + 40 ° C<br>Verpackung: Glasrundkörper mit dichtem Schraubverschluß | | |
|---|---|---|
| Zusammensetzung in mg | Standardrezeptur | Erfindungsgemäß hergestellte Tabletten entspr. Beispiel 5 |
| Ramipril-Substanz ungecoatet | 2,50 | - |
| Ramipril-Substanz 87 %ig*<br>* enthält 13 % HPMC als Filmhülle | - | 2,87 |
| Mikrokristalline Cellulose<br>Mannit freifließend<br>Na-Stearylfumarat | 47,00<br>49,50<br>1,00 | 47,00<br>49,13<br>1,00 |
| Tablettengewicht | 100,00 | 100,00 |
| Preßkraft | 10 000 N | 10 000 N |
| Zersetzung zum Diketopiperazinabbauprodukt in % | 12,72 | 1,87 |

Der Nachweis, daß eine dünnere Beschichtung von Ramipril selbst nach einer Langzeitbelastung noch effektiv ist, geht aus der folgenden Tabelle 2 hervor.

Tabelle 2

| Ramipril-Tabletten zu 2,5 mg<br>Stabilitätsvergleich<br>Belastungsart: 12 Monate + 40 ° C<br>Verpackung: Glasrundkörper mit dichtem Schraubverschluß | | |
|---|---|---|
| Zusammensetzung in mg | Standardrezeptur | Erfindungsgemäß hergestellte Tabletten entspr. Beispiel 6 |
| Ramipril-Substanz ungecoatet | 2,50 | - |
| Ramipril-Substanz 94 %ig*<br>* enthält 6 % HPMC als Filmhülle | - | 2,66 |
| Mikrokristalline Cellulose<br>Mannit freifließend<br>Na-Stearylfumarat | 25,00<br>71,50<br>1,00 | 25,00<br>71,34<br>1,00 |
| Tablettengewicht | 100,00 | 100,00 |
| Preßkraft beim Tablettieren | 10 000 N | 10 000 N |
| Zersetzung zum Diketopiperazinabbauprodukt in % | 25,34 | 5,97 |

Bei der Stabilisierung durch Zusatz eines Puffers wird letzterer entweder dem Wirkstoff oder dem umhüllten Wirkstoff zugesetzt, der Wirkstoff oder der umhüllte Wirkstoff wird dabei mit einer Pufferlösung granuliert oder er befindet sich in der Dispersion oder Lösung der Polymersubstanz, wenn beide Stabilisierungsarten gleichzeitig verwendet werden.

In der Zubereitung, wie z.B. einer Tablette, stellt sich in Gegenwart von Feuchtigkeit, wie z.B. Luftfeuchtigkeit oder Wasser ein pH-Wert zwischen 5,5 und 8,0 ein.

Als Puffersubstanzen sind z.B. geeignet: Natriumdihydrogenphosphat dihydrat, tri-Natriumcitrat dihydrat, Natriumcarbonat, Natriumhydrogencarbonat, Tris-(hydroxymethyl)-aminomethan.

Es ist vorteilhaft, wenn die Puffersubstanz als wäßrige Lösung verwendet wird, indem man entweder

Wirksubstanz in einem geeigneten Mischer, Kneter, Mischer-Granulator gleichmäßig befeuchtet und dann granuliert und trocknet oder in der Wirbelschicht besprüht und auf diese Weise sprühgranuliert. Es kann aber auch eine Mischung von Wirkstoff und Puffersubstanz auf die beschriebene Weise mit Wasser granuliert werden.

Als besonders vorteilhaft hat es sich erwiesen, wenn der sich durch Pufferzusatzt einstellende stabilisierende Effekt mit einer Schutzumhüllung der Wirkstoff-Partikel durch polymere Filmbildner kombiniert wird.

Dies geschieht am vorteilhaftesten in der Weise, daß die Puffersubstanz bereits in dem zum Umhüllen der Partikel vorgesehenen Medium gelöst und zusammen mit dem polymeren Filmbildner auf die Oberfläche des Wirkstoffs aufgebracht wird. Dabei kommt die zur Umhüllung der Partikel beschriebene Beschichtungstechnik zur Anwendung.

Der stabilisierende Effekt von Puffersubstanzen wird durch folgenden tabellarischen Vergleich (Tabelle 3) verdeutlicht.

Tabelle 3

| Ramipril-Tabletten zu 2,5 mg<br>Stabilitätsvergleich/stabilisierender Einfluß von Puffersubstanzen<br>Belastungsart: 3 Monate + 40 °C<br>Verpackung: Glasrundkörper mit dichtem Schraubverschluß | | |
|---|---|---|
| Zusammensetzung in mg | Standardrezeptur | Erfindungsgemäß hergestellte Tabletten entspr. Beispiel 7 |
| Ramipril-Substanz | 2,5 | 2,5 |
| Tris-(hydroxymethyl)-aminomethan<br>Pregelatineisierte Stärke<br>Mikrokristalline Cellulose<br>Na-Stearlyfumarat | -<br>51,5<br>45,0<br>1,0 | 2,5<br>49,0<br>45,0<br>1,0 |
| Tablettengewicht | 100,0 | 100,0 |
| Preßkraft beim Tablettieren | 10 000 N | 10 000 N |
| pH-Wert nach Suspendieren mit Wasser | 5.4 | 6,9 |
| Zersetzung zum Diketopiperazinabbauprodukt in % | 7,1 | 0,6 |

## Anwendungsbeispiele

### Beispiel 1

**Herstellung von stabilisierter Ramipril-Substanz**

87 Gewichtsteile Ramipril-Substanz werden in einer Wirbelschichtapparatur mit 13 Gewichtsteilen Hydroxypropylmethylcellulose, im folgenden HPMC genannt, als 5 %ige wäßrige Lösung granuliert. Geeignet sind z.B. die Typen Pharmacoat® 606 oder Methocel® E5 Premium. Das Verfahren verläuft in zwei Abschnitten, in dem zunächst die Ramipril-Substanz mit der Hälfte der HPMC-Lösung granuliert und anschließend mit der zweiten Hälfte der 5 %igen wäßrigen HPMC-Lösung gecoatet wird.

Die Trocknungstemperatur beträgt etwa 50 °C. Die gecoatete Ramipril-Substanz kann im Gemisch mit Hilfsstoffen in Kapseln abgefüllt oder direkt, ohne weitere Granulationsschritte zu Tabletten verpreßt werden.

### Beispiel 2

**Herstellung von stabilisierter Ramipril-Substanz**

94 Gewichtsteile Ramipril-Substanz werden in einem geeigneten Kneter, Mischer oder Mischer-Granulator mit 6 Gewichtsteilen HMPC als 10 %ige wäßrige Lösung so lange dispergiert, bis eine gleichmäßig befeuchtete Masse entstanden ist. Die feuchte Masse wird durch ein Sieb mit einer Maschenweite von 1,2

mm passiert und anschließend bei ca. 40 ° C getrocknet. Die getrockneten Agglomerate werden nochmals durch ein Sieb mit einer Maschenweite von 0,5 bis 1 mm passiert. Das gebrauchsfertige Ramipril-Granulat kann zur Herstellung von Kapseln oder Tablettten eingesetzt werden.

**Beispiel 3**

**Herstellung von stabilisierter Ramipril-Substanz**

1 Gewichtsteil Ramipril-Substanz und 1 Gewichtsteil Tris-(hydroxymethyl)-aminomethan-Puffersubstanz werden in einem geeigneten Mischer oder Mischer-Granulator gemischt und dann mit so viel gereinigtem Wasser befeuchtet, bis eine gleichmäßige benetzte Masse entstanden ist.

Die feuchte Masse wird auf die in Beispiel 2 beschriebene Weise granuliert.

**Beispiel 4**

**Herstellung von stabilisierter Ramipril-Substanz**

94 Gewichtsteile Ramipril-Substanz, 6 Gewichtsteile Polyvinylpyrrolidon (z.B. Kollidon® K25), 18,8 Gewichtsteile Natriumcarbonat werden in einem geeigneten Mischer oder Mischer-Granulator gemischt und dann mit soviel gereinigtem Wasser befeuchtet, bis eine gleichmäßig benetzte Masse entstanden ist. Die feuchte Masse wird auf die in Beispiel 2 beschriebene Weise granuliert.

**Beispiel 5**

**Herstellung von 10 000 Ramipril-Tabletten zu 2,5 mg**

28,7 g Ramipril 87 %ig (enthält 13 % HPMC als Filmhülle gemäß Beispiel 1), 470 g mikrokristalline Cellulose und 491,3 g Mannit freifließend werden gemischt. Dieser Mischung werden in einem zweiten Schritt 10 g Natriumstearylfumarat zugemischt. 1 kg der so hergestellten Mischung werden direkt, ohne weitere Granulationsschritte, zu Tabletten mit einem Endgewicht von 100 mg verpreßt.

**Beispiel 6**

**Herstellung von 10 000 Ramipril-Tabletten zu 2,5 mg**

26,6 g Ramipril 94 %ig (enthält 6 % HPMC als Filmhülle gemäß Beispiel 2), 250 g mikrokristalline Cellulose und 713,4 g Mannit freifließend werden gemischt. Dieser Mischung werden in einem zweiten Schritt 10 g Natriumstearylfumarat zugemischt.

1 kg der so hergestellten Mischung werden direkt, ohne weitere Granulationsschritte, zu Tabletten mit einem Endgewicht von 100 mg verpreßt.

**Beispiel 7**

**Herstellung von 10 000 Ramipril-Tabletten zu 2,5 mg**

50 g Ramipril 50 %ig, hergestellt gemäß Beispiel 3, 450 g mikrokristalline Cellulose und 490 g pregelatinisierte Stärke werden gemischt. Dieser Mischung werden in einem zweiten Schritt 10 g Natriumstearylfumarat zugemischt.

1 kg der so hergestellten Mischung werden direkt, ohne weitere Granulationsschritte, zu Tabletten mit einem Endgewicht von 100 mg verpreßt.

**Beispiel 8**

**Herstellung von 10 000 Ramipril-Tabletten zu 5 mg**

63 g Ramipril stabilisiert gemäß Beispiel 4, 250 g mikrokristalline Cellulose und 667 g Mannit freifließend werden gemischt. Dieser Mischung werden in einem zweiten Schritt 20 g Natriumstearylfumarat zugemischt.

1 kg dieser Mischung werden direkt, ohne weitere Granulationsschritte, zu Tabletten mit einem Endgewicht von 100 mg verpreßt.

**Beispiel 9**

**Herstellung stabilisierter Enalapril-Substanz**

85 Gewichtsteile Enalaprilhydrogenmaleat werden in einer Wirbelschichtapparatur mit 15 Gewichtsteilen Hydroxypropylmethylcellulose (HPMC) als 5 %ige wäßrige Lösung auf die in Beispiel 1 angegebene Weise granuliert. Die gecoatete Enalapril-Substanz kann im Gemisch mit Hilfsstoffen in Kapseln abgefüllt oder direkt, ohne weitere Granulationsschritte zu Tabletten verpreßt werden.

**Beispiel 10**

**Herstellung stabilisierter Enalapril-Substanz**

90 Gewichtsteile Enalaprilhydrogenmaleat werden in einem geeigneten Kneter, Mischer oder Mischer-Granulator mit 10 Gewichtsteilen HPMC als wäßrige Lösung so lange dispergiert, bis eine gleichmäßig befeuchtete Masse entstanden ist. Die feuchte Enalapril-Masse wird auf die in Beispiel 2 beschriebene Weise granuliert.
Das gebrauchsfertige schutzumhüllte Enalapril-Granulat kann zur Herstellung von Kapseln oder Tabletten eingesetzt werden.

**Beispiel 11**

**Herstellung von 10 000 Enalapril-Tabletten zu 2,5 mg**

29,4 g Enalaprilhydrogenmaleat 85 %ig (enthält 15 % HPMC als Filmhülle gemäß Beispiel 9), 480g mikrokristalline Zellulose und 480,6 g modifizierte Stärke freifließend werden gemischt. Dieser Mischung werden in einem zweiten Schritt 10 g Natriumstearylfumarat zugemischt. 1 kg dieser Mischung werden direkt, ohne weitere Granulationsschritte, zu Tabletten mit einem Endgewicht von 100 mg verpreßt.

**Beispiel 12**

**Herstellung von 10 000 Enalapril-Tabletten zu 10 mg**

111,1 g Enalaprilhydrogenmaleat 90 %ig (enthält 10 % HPMC als Filmhülle gemäß Beispiel 10), 480 g mikrokristalline Zellulose und 398,9 g modifizierte Stärke freifließend werden gemischt. Dieser Mischung werden in einem zweiten Schritt 10 g Natriumstearylfumarat zugemischt. 1 kg dieser Mischung werden direkt, ohne weitere Granulationsschritte, zu Tabletten mit einem Endgewicht von 100 mg verpreßt.

**Patentansprüche**
**Patentansprüche für folgende Vertragsstaaten : AT, BE, CH, DE, FR, GB, IT, LI, LU, NL, SE**

**1.** Stabile, gepreßte pharmazeutische Zubereitung enthaltend eine Verbindung der Formel I

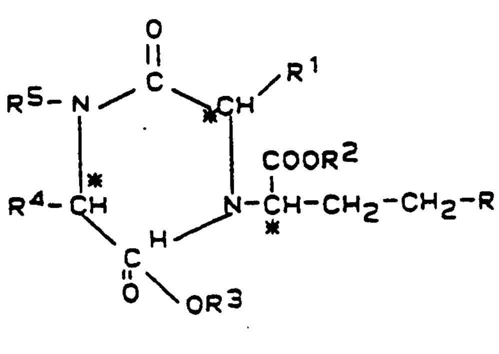

worin

R Wasserstoff, $C_1$-$C_4$-Alkyl oder Phenyl ist

$R^1$ $C_1$-$C_4$-Alkyl oder

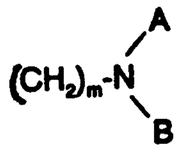 ,

worin m 1, 2, 3 oder 4 ist und A und B gleich oder verschieden sind und Wassestoff oder $C_1$-$C_4$-Alkyl bedeuten, darstellt,

$R^2$ Wasserstoff, $C_1$-$C_4$-Alkyl oder Benzyl ist,

$R^3$ Wasserstoff oder $C_1$-$C_4$-Alkyl ist und

$R^4$ und $R^5$ zusammen mit den sie tragenden Atomen ein heterocyclisches, mono-, bi- oder tricyclisches hydriertes oder teilhydriertes Ringsystem mit 1 Stickstoffatom und 4 bis 15 Ringkohlenstoffatomen, das gegebenenfalls mono- oder disubstituiert ist mit $C_1$-$C_4$-Alkoxy, bedeuten, oder deren physiologisch verträgliche Salze,

dadurch gekennzeichnet, daß eine Verbindung der Formel I oder ein physiologisch verträgliches Salz einer Verbindung der Formel I zur Stabilisierung vor dem Verpressen

a) mit einer polymeren, physiologisch verträglichen Schutzhülle umhüllt wird oder

b) mit einem physiologisch vertraglichen Puffer, der sicherstellt, daß sich in einer pharmazeutischen Zubereitung in Gegenwart Von Feuchtigkeit ein pH-Wert im schwach sauren bis schwach alkalischen Bereich einstellt, vermischt wird, wobei Natriumhydrogencarbonat als Puffer ausgeschlossen ist, oder

c) mit einem physiologisch verträglichen Puffer, der sicherstellt, daß sich in einer pharmazeutischen Zubereitung in Gegenwart von Feuchtigkeit ein pH-Wert im schwach sauren bis schwach alkalischen Bereich einstellt, vermischt und mit einer polymeren, physiologisch verträglichen Schutzhülle umhüllt wird,

wobei im Falle der Stabilisierung gemäß b) mit Alkali- oder Erdalkalicarbonat die Zubereitung frei von Zucker ist.

**2.** Stabile, gepreßte pharmazeutische Zubereitung gemäß Anspruch 1 enthaltend Ramipril, dadurch gekennzeichnet, daß Ramipril zur Stabilisierung vor dem verpressen mit einer physiologisch verträglichen polymeren Schutzhülle umhüllt wird.

**3.** Verfahren zur Herstellung von stabilen, gepreßten pharmazeutischen Zubereitungen gemäß Anspruch 1, dadurch gekennzeichnet, daß man eine Verbindung der Formel I oder ein physiologisch verträgliches Salz einer Verbindung der Formel I nach üblichen Verfahren mit einem physiologisch verträglichen polymeren Schutzfilm überzieht und/oder daß man einen physiologisch verträglichen Puffer zusetzt und anschließend verpreßt.

**4.** Verfahren gemäß Anspruch 3, dadurch gekennzeichnet, daß man die Wirksubstanz mit einem physiologisch verträglichen polymeren Schutzfilm überzieht und, gegebenenfalls unter Zusatz üblicher Hilfsstoffe, verpreßt.

**5.** Verfahren gemäß Anspruch 3, dadurch gekennzeichnet, daß man die Umgebung des Wirkstoffs durch Zusatz von Puffersubstanzen auf einen pH-Bereich von 5,5 bis 8, vorzugsweise 6,5 bis 7, einstellt.

**6.** Ramipril enthaltende Zubereitung erhältlich nach Verfahren gemäß Anspruch 4, dadurch gekennzeichnet, daß der Gewichtsanteil der Schutzumhüllung 3 bis 25 %, vorzugsweise 5 bis 15 %, bezogen auf den Wirkstoff, beträgt.

**7.** Ramipril enthaltende Zubereitung erhältlich nach Verfahren gemäß Anspruch 5.

**8.** Verwendung von stabilisierten Wirkstoffen gemäß Anspruch 1 zur Herstellung von pharmazeutischen Zubereitungen.

**Patentansprüche für folgende Vertragsstaaten : ES, GR**

1. Verfahren zur Herstellung stabiler, gepreßter pharmazeutischer Zubereitungen enthaltend eine Verbindung der Formel I

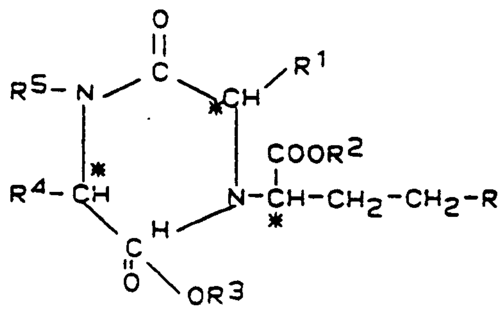

worin

R Wasserstoff, $C_1$-$C_4$-Alkyl oder Phenyl ist,

$R^1$ $C_1$-$C_4$-Alkyl oder

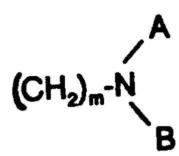 ,

worin m 1, 2, 3 oder 4 ist und A und B gleich oder verschieden sind und Wassestoff oder $C_1$-$C_4$-Alkyl bedeuten, darstellt,

$R^2$ Wasserstoff, $C_1$-$C_4$-Alkyl oder Benzyl ist,

$R^3$ Wasserstoff oder $C_1$-$C_4$-Alkyl ist und

$R^4$ und $R^5$ zusammen mit den sie tragenden Atomen ein heterocyclisches, mono-, bi- oder tricyclisches hydriertes oder teilhydriertes Ringsystem mit 1 Stickstoffatom und 4 bis 15 Ringkohlenstoffatomen, das gegebenenfalls mono- oder disubstituiert ist mit $C_1$-$C_4$-Alkoxy, bedeuten, oder deren physiologisch verträgliche Salze,

dadurch gekennzeichnet, daß man eine Verbindung der Formel I oder ein physiologisch verträgliches Salz einer Verbindung der Formel I

a) mit einer polymeren, physiologisch verträglichen Schutzhülle umhüllt, oder

b) mit einem physiologisch verträglichen Puffer, der sicherstellt, daß sich in einer pharmazeutischen Zubereitung in Gegenwart von Feuchtigkeit ein pH-Wert im schwach sauren bis schwach alkalischen Bereich einstellt, vermischt, wobei Natriumhydrogencarbonat als Puffer ausgeschlossen ist, oder

c) mit einem physiologisch verträglichen Puffer, der sicherstellt, daß sich in einer pharmazeutischen Zubereitung in Gegenwart von Feuchtigkeit ein pH-Wert im schwach sauren bis schwach alkalischen Bereich einstellt, vermischt und mit einer polymeren, physiologisch verträglichen Schutzhülle umhüllt

und anschließend die so behandelte Verbindung verpreßt, wobei im Falle der Stabilisierung gemäß b) mit Alkali- oder Erdalkalicarbonat die erhaltene Zubereitung frei von Zucker ist.

2. Verfahren gemäß Anspruch 1, dadurch gekennzeichnet, daß man Ramipril als Verbindung der Formel I mit einer physiologisch verträglichen polymeren Schutzhülle umhüllt.

3. Verfahren gemäß Anspruch 1, dadurch gekennzeichnet, daß man die Wirksubstanz mit einem physiologisch verträglichen polymeren Schutzfilm überzieht und, gegebenenfalls unter Zusatz üblicher Hilfsstoffe, verpreßt.

4. Verfahren gemaß Anspruch 1, dadurch gekennzeichnet, daß man die Umgebung des Wirkstoffs durch

Zusatz von Puffersubstanzen auf einen pH-Bereich von 5,5 bis 8, vorzugsweise 6,5 bis 7, einstellt.

**5.** Ramipril enthaltende Zubereitung erhältlich nach Verfahren gemäß Anspruch 3, dadurch gekennzeichnet, daß der Gewichtsanteil der Schutzumhüllung 3 bis 25 %, vorzugsweise 5 bis 15 %, bezogen auf den Wirkstoff, beträgt.

**6.** Ramipril enthaltende Zubereitung erhältlich nach Verfahren gemäß Anspruch 4.

**7.** Verwendung von stabilisierten Wirkstoffen gemäß Anspruch 1 zur Herstellung von pharmazeutischen Zubereitungen.

**Claims**
**Claims for the following Contracting States : AT, BE, CH, DE, FR, GB, IT, LI, LU, NL, SE**

**1.** A stable, compressed pharmaceutical formulation containing a compound of the formula I

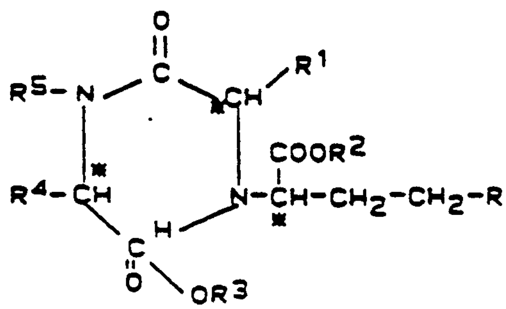

in which

| | |
|---|---|
| R | is hydrogen, $C_1$-$C_4$-alkyl or phenyl, |
| $R^1$ | represents $C_1$-$C_4$-alkyl or |

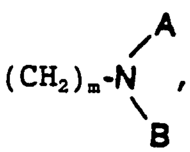

in which m is 1, 2, 3 or 4, and A and B are identical or different and denote hydrogen or $C_1$-$C_4$-alkyl,

| | |
|---|---|
| $R^2$ | is hydrogen, $C_1$-$C_4$-alkyl or benzyl, |
| $R^3$ | is hydrogen or $C_1$-$C_4$-alkyl, and |
| $R^4$ and $R^5$ | denote, together with the atoms carrying them, a heterocyclic, mono-, bi- or tricyclic hydrogenated or partially hydrogenated ring system which has 1 nitrogen atom and 4 to 15 ring carbon atoms and which is optionally mono- or disubstituted by $C_1$-$C_4$-alkoxy, or the physiologically tolerated salts thereof, |

wherein, for stabilization before compression, a compound of the formula I or a physiologically tolerated salt of a compound of the formula I

a) is coated with a polymeric, physiologically tolerated protective coating or

b) is mixed with a physiologically tolerated buffer which ensures that a pH in the weakly acid to weakly alkaline range is set up in a pharmaceutical formulation in the presence of moisture, where sodium bicarbonate is excepted as buffer, or

c) is mixed with a physiologically tolerated buffer which ensures that a pH in the weakly acid to weakly alkaline range is set up in a pharmaceutical formulation in the presence of moisture, and is coated with a polymeric, physiologically tolerated protective coating,

where, in the case of stabilization according to b) with alkali metal or alkaline earth metal carbonate, the formulation is free of sugar.

**2.** A stable, compressed pharmaceutical formulation as claimed in claim 1 containing ramipril, wherein ramipril is coated with a physiologically tolerated polymeric protective coating for stabilization before compression.

**3.** A process for the manufacture of stable, compressed pharmaceutical formulations as claimed in claim 1, which comprises coating a compound of the formula I or a physiologically tolerated salt of a compound of the formula I by customary processes with a physiologically tolerated polymeric protective film, and/or adding a physiologically tolerated buffer and subsequently compressing.

**4.** The process as claimed in claim 3, wherein the active substance is coated with a physiologically tolerated polymeric protective film and compressed, where appropriate with the addition of customary auxiliaries.

**5.** The process as claimed in claim 3, wherein the environment of the active substance is adjusted to a pH range from 5.5 to 8, preferably 6.5 to 7, by adding buffer substances.

**6.** A formulation which contains ramipril and can be obtained by the process as claimed in claim 4, wherein the proportion by weight of the protective coating is 3 to 25%, preferably 5 to 15%, relative to the active substance.

**7.** A formulation which contains ramipril and can be obtained by the process as claimed in claim 5.

**8.** The use of stabilized active substances as claimed in claim 1 for the preparation of pharmaceutical formulations.

**Claims for the following Contracting States : ES, GR**

**1.** A process for the preparation of stable, compressed pharmaceutical formulations containing a compound of the formula I

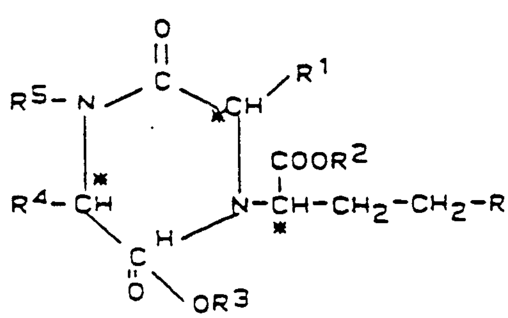

in which

| | |
|---|---|
| R | is hydrogen, $C_1$-$C_4$-alkyl or phenyl, |
| $R^1$ | represents $C_1$-$C_4$-alkyl or |

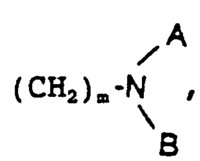

in which m is 1, 2, 3 or 4, and A and B are identical or different and denote hydrogen or $C_1$-$C_4$-alkyl,

| | |
|---|---|
| $R^2$ | is hydrogen, $C_1$-$C_4$-alkyl or benzyl, |
| $R^3$ | is hydrogen or $C_1$-$C_4$-alkyl |
| $R^4$ and $R^5$ | denote, together with the atoms carrying them, a heterocyclic, mono-, bi- or tricyclic hydrogenated or partially hydrogenated ring system which has 1 nitrogen atom and 4 |

to 15 ring carbon atoms and which is optionally mono- or disubstituted by $C_1$-$C_4$-alkoxy, or the physiologically tolerated salts thereof,

which comprises a compound of the formula I or a physiologically tolerated salt of a compound of the formula I

a) being coated with a polymeric, physiologically tolerated protective coating or

b) being mixed with a physiologically tolerated buffer which ensures that a pH in the weakly acid to weakly alkaline range is set up in a pharmaceutical formulation in the presence of moisture, where sodium bicarbonate is excepted as buffer, or

c) being mixed with a physiologically tolerated buffer which ensures that a pH in the weakly acid to weakly alkaline range is set up in a pharmaceutical formulation in the presence of moisture, and being coated with a polymeric, physiologically tolerated protective coating,

and the compound treated in this way is then compressed, where, in the case of stabilization according to b) with alkali metal or alkaline earth metal carbonate, the resulting formulation is free of sugar.

**2.** The process as claimed in claim 1, wherein ramipril as compound of the formula I is coated with a physiologically tolerated polymeric protective coating.

**3.** The process as claimed in claim 1, wherein the active substance is coated with a physiologically tolerated polymeric protective film and is compressed, where appropriate with the addition of customary auxiliaries.

**4.** The process as claimed in claim 1, wherein the environment of the active substance is adjusted to a pH range from 5.5 to 8, preferably 6.5 to 7, by adding buffer substances.

**5.** A formulation which contains ramipril and can be obtained by the process as claimed in claim 3, wherein the proportion by weight of the protective coating is 3 to 25%, preferably 5 to 15%, relative to the active substance.

**6.** A formulation which contains ramipril and can be obtained by the process as claimed in claim 4.

**7.** The use of stabilized active substances as claimed in claim 1 for the manufacture of pharmaceutical formulations.

**Revendications**

**Revendications pour les Etats contractants suivants : AT, BE, CH, DE, FR, GB, IT, LI, LU, NL, SE**

**1.** Préparation pharmaceutique stable, comprimée, contenant un composé de formule I

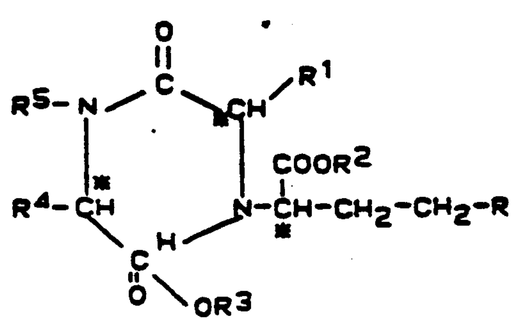

I

dans laquelle

R est un atome d'hydrogène, un groupe alkyle en $C_1$-$C_4$ ou un groupe phényle,

$R^1$ représente un groupe alkyle en $C_1$-$C_4$ ou

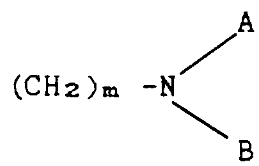

où m est égal à 1, 2, 3 ou 4, et A et B sont identiques ou différents et signifient un atome d'hydrogène ou un groupe alkyle en $C_1$-$C_4$,
$R^2$ est un atome d'hydrogène, un groupe alkyle en $C_1$-$C_4$ ou un groupe benzyle,
$R^3$ est un atome d'hydrogène ou un groupe alkyle en $C_1$-$C_4$, et
$R^4$ et $R^5$ représentent, conjointement avec les atomes qui les portent, un système hétérocyclique, mono-, bi- ou tricyclique, hydrogéné ou partiellement hydrogéné, comportant un atome d'azote et 4 à 15 atomes de carbone cyclisés, et éventuellement mono- ou dissubstitué par un ou des groupe(s) alkoxy en $C_1$-$C_4$,
ou des sels physiologiquement acceptables de celui-ci,
caractérisée en ce que, afin d'assurer la stabilisation, préalablement à la compression, un composé de formule I, ou un sel physiologiquement acceptable d'un composé de formule I,

a) est enrobé d'une enveloppe protectrice polymère, physiologiquement acceptable, ou

b) est mélangé avec un tampon physiologiquement acceptable qui assure que, dans une préparation pharmaceutique en présence d'humidité, le pH s'établit dans une zone faiblement acide à faiblement alcaline, ce qui exclut l'hydrogénocarbonate de sodium comme tampon, ou

c) est mélangé avec un tampon physiologiquement acceptable qui assure que, dans une préparation pharmaceutique en présence d'humidité, le pH s'établit dans une zone faiblement acide à faiblement alcaline, et est enrobé d'une enveloppe protectrice polymère, physiologiquement acceptable,

la préparation étant dépourvue de sucre dans le cas d'une stabilisation selon b) avec un carbonate alcalin ou alcalino-terreux.

**2.** Préparation pharmaceutique stable, comprimée, selon la revendication 1, contenant du ramipril, caractérisée en ce que, afin d'assurer la stabilisation, préalablement à la compression, on enrobe le ramipril d'une enveloppe protectrice polymère, physiologiquement acceptable.

**3.** Procédé pour préparer des compositions pharmaceutiques stables, comprimées, selon la revendication 1, caractérisé en ce qu'on recouvre, selon des procédés usuels, un composé de formule I, ou un sel physiologiquement acceptable d'un composé de formule I, d'un film protecteur polymère, physiologiquement acceptable et/ou on ajoute un tampon physiologiquement acceptable et on comprime ensuite.

**4.** Procédé selon la revendication 3, caractérisé en ce qu'on recouvre la substance active d'un film protecteur polymère, physiologiquement acceptable, et on comprime, en ajoutant éventuellement des adjuvants usuels.

**5.** Procédé selon la revendication 3, caractérisé en ce qu'on ajuste le pH du milieu contenant le principe actif dans une zone allant de 5,5 à 8, de préférence allant de 6,5 à 7, par addition de substances tampons.

**6.** Préparation contenant du ramipril qu'on peut obtenir au moyen du procédé selon la revendication 4, caractérisée en ce que la proportion pondérale de l'enrobage protecteur est de 3 à 25%, de préférence de 5 à 15%, par rapport au poids du principe actif.

**7.** Préparation contenant du ramipril qu'on peut obtenir au moyen du procédé selon la revendication 5.

**8.** Utilisation de principes actifs stabilisés selon la revendication 1 pour élaborer des préparations pharmaceutiques.

**Revendications pour les Etats contractants suivants : ES, GR**

**1.** Procédé pour élaborer une préparation pharmaceutique stable, comprimée, contenant un composé de formule I

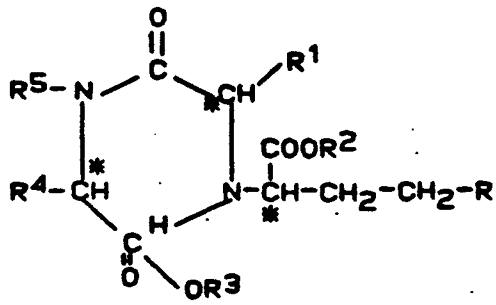

I

dans laquelle
R est un atome d'hydrogène, un groupe alkyle en $C_1$-$C_4$ ou un groupe phényle,
$R^1$ représente un groupe alkyle en $C_1$-$C_4$ ou

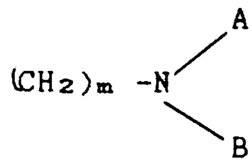

où m est égal à 1, 2, 3 ou 4, et A et B sont identiques ou différents et signifient un atome d'hydrogène ou un groupe alkyle en $C_1$-$C_4$,
$R^2$ est un atome d'hydrogène, un groupe alkyle en $C_1$-$C_4$ ou un groupe benzyle,
$R^3$ est un atome d'hydrogène ou un groupe alkyle en $C_1$-$C_4$, et
$R^4$ et $R^5$ représentent, conjointement avec les atomes qui les portent, un système hétérocyclique, mono-, bi- ou tricyclique, hydrogéné ou partiellement hydrogéné, comportant un atome d'azote et 4 à 15 atomes de carbone cyclisés, et éventuellement mono- ou dissubstitué par un ou des groupe(s) alkoxy en $C_1$-$C_4$,
ou des sels physiologiquement acceptables de celui-ci,
caractérisé en ce qu'un composé de formule I, ou un sel physiologiquement acceptable d'un composé de formule I,

a) est enrobé d'une enveloppe protectrice polymère, physiologiquement acceptable, ou
b) est mélangé avec un tampon physiologiquement acceptable qui assure que, dans une préparation pharmaceutique en présence d'humidité, le pH s'établit dans une zone faiblement acide à faiblement alcaline, ce qui exclut l'hydrogénocarbonate de sodium comme tampon, ou
c) est mélangé avec un tampon physiologiquement acceptable qui assure que, dans une préparation pharmaceutique en présence d'humidité, le pH s'établit dans une zone faiblement acide à faiblement alcaline, et est enrobé d'une enveloppe protectrice polymère, physiologiquement acceptable,

et on comprime ensuite le composé ainsi traité, la préparation obtenue étant dépourvue de sucre dans le cas d'une stabilisation selon b) avec un carbonate alcalin ou alcalino-terreux.

2. Procédé selon la revendication 1, caractérisé en ce qu'on enrobe du ramipril, comme composé de formule I, d'une enveloppe protectrice polymère, physiologiquement acceptable.

3. Procédé selon la revendication 1, caractérisé en ce qu'on recouvre la substance active d'un film protecteur polymère, physiologiquement acceptable, et on comprime, en ajoutant éventuellement des adjuvants usuels.

4. Procédé selon la revendication 1, caractérisé en ce qu'on ajuste le pH du milieu contenant le principe actif dans une zone allant de 5,5 à 8, de préférence allant de 6,5 à 7, par addition de substances tampons.

5. Préparation contenant du ramipril qu'on peut obtenir au moyen du procédé selon la revendication 3, caractérisée en ce que la proportion pondérale de l'enrobage protecteur est de 3 à 25%, de préférence de 5 à 15%, par rapport au poids du principe actif.

6. Préparation contenant du ramipril qu'on peut obtenir au moyen du procédé selon la revendication 4.

7. Utilisation de principes actifs stabilisés selon la revendication 1 pour la fabrication de préparations pharmaceutiques.